## Europäisches Patentamt
## European Patent Office
## Office européen des brevets

(19)

(11) Veröffentlichungsnummer: **0 063 270**
**B1**

(12)

# EUROPÄISCHE PATENTSCHRIFT

(45) Veröffentlichungstag der Patentschrift:
02.01.85

(51) Int. Cl.³: **C 07 C 143/58,** C 07 C 139/00

(21) Anmeldenummer: **82102728.1**

(22) Anmeldetag: **31.03.82**

(54) Verfahren zur Herstellung von p-Sulfanilsäure.

(30) Priorität: **11.04.81 DE 3114832**

(43) Veröffentlichungstag der Anmeldung:
**27.10.82 Patentblatt 82/43**

(45) Bekanntmachung des Hinweises auf die Patenterteilung:
**02.01.85 Patentblatt 85/1**

(84) Benannte Vertragsstaaten:
**CH DE FR GB LI**

(56) Entgegenhaltungen:
**DE - A - 2 439 297**

**HOUBEN WEYL "Methoden der organischen Chemie" 4. Auflage, Band IX 1955, GEORG THIEME VERLAG, Stuttgart
Chemical Abstracts Band 48, Nr. 13 10. Juli 1954 Columbus, Ohio, USA Z. SKROWACZEWSKA "Sulfonation of aromatic amines" Spalten 7568i bis 7569i**

(73) Patentinhaber: **BAYER AG, Konzernverwaltung RP Patentabteilung, D-5090 Leverkusen 1 Bayerwerk (DE)**

(72) Erfinder: **Emde, Herbert, Dr.,
Andreas-Gryphiusstrasse 7, D-5000 Koeln 80 (DE)**
Erfinder: **Blank, Heinz Ulrich, Dr., Am Geusfelde 35, D-5068 Odenthal (DE)**
Erfinder: **Schnegg, Peter, Dr., Heidbergerstrasse 44, D-5068 Odenthal (DE)**

## Beschreibung

p-Sulfanilsäure (4-Amino-benzolsulfonsäure) ist ein wertvolles Zwischenprodukt, das in der organischen Synthese weite Anwendung findet (Ullmanns Enzyklopädie der technischen Chemie, 3. Auflage, Band 16, Seite 561, Urban und Schwarzenberg, München/Berlin 1965), wobei als Anwendungsgebiete besonders die Bildung von Azofarbstoffen sowie von optischen Aufhellern hervorgehoben sei.

Es ist bereits bekannt, p-Sulfanilsäure durch Umsetzung von Anilin und Schwefelsäure nach dem sogenannten Backverfahren in einem indifferenten Lösungsmittel bei Entfernung des Reaktionswassers aus dem Ansatz herzustellen (Ind. Eng. Chem. 35, 321 (1943), Ind. Eng. Chem. 42, 1746 (1950), Khim. Prom. 41, 104 (1965), DE-PS 549 136, Russ. Patent 667 550; N. N. Woroshzow, Grundlagen der Synthese von Zwischenprodukten und Farbstoffen, Akademie-Verlag, Berlin 1966, Seite 72). Als Lösungsmittel sind hierin Kerosinfraktionen, Petroleum und Polychloraromaten beschrieben. In allen Verfahren wird die Schwefelsäure im äquimolaren Verhältnis oder im Überschuß zum Anilin eingesetzt. Die genannten Verfahrensvarianten weisen verschiedene Nachteile auf. So ist bei Verwendung von Kerosin als Lösungsmittel ein hohes Verhältnis von Lösungsmittel zu dem als Zwischenprodukt auftretenden Anilinsulfat erforderlich, da es sonst zu Verbackungen und Teerbildung kommt. Beim Einsatz von Petroleum als Verdünnungsmittel kann dieses nicht unbehandelt zurückgeführt werden, da keine Phasentrennung zwischen dem abdestillierten Wasser und dem mitdestillierten Lösungsmittel eintritt. Vielmehr muß das abdestillierte Gemisch durch frisches Petroleum ersetzt werden. Diese Schwierigkeiten treten zwar bei der Verwendung von Polychloraromaten als Lösungsmittel nicht auf, dagegen werden in solchen Fällen nachteilig lange Reaktionszeiten erforderlich und weiterhin werden nachteilig große Verdünnermengen sowie die mangelnde Qualität der Endprodukte beschrieben, die einen eigenen Reinigungsschritt erforderlich macht.

Es wurde nun ein Verfahren zur Herstellung von p-Sulfanilsäure nach dem sogenannten Backverfahren durch Umsetzung von Aniliniumhydrogensulfat bei erhöhter Temperatur in einem inerten Lösungsmittel gefunden, das dadurch gekennzeichnet ist, daß die Umsetzung in Gegenwart eines Überschusses von Anilin, der über das äquimolare Verhältnis von Anilin und Schwefelsäure hinausgeht, vorgenommen wird und daß die Entfernung des Reaktionswassers aus dem Reaktionsgemisch mindestens teilweise bei 180 bis 280° C, gegebenenfalls unter Druck, durchgeführt wird.

Erfindungsgemäß wird ein Überschuß von Anilin, der über das äquimolare Verhältnis von Anilin und Schwefelsäure hinausgeht, eingesetzt. Dieser Überschuß beträgt mindestens 0,01 Mol-%, beispielsweise 0,01 bis 30 Mol-%. Bevorzugt ist ein Anilinüberschuß von 0,05 bis 10 Mol-%, besonders bevorzugt ein Anilinüberschuß von 0,5 bis 2 Mol-%.

Als Lösungsmittel können beispielsweise alkyl- und/oder halogensubstituierte Aromaten eingesetzt werden. Hierunter seien beispielsweise ein Benzol, Naphthalin, Anthracen oder Diphenyl verstanden, das bis zu 4 Alkylgruppen und/oder bis zu 4 Halogenatome, wie Chlor oder Brom, als Substituenten tragen kann, wobei die Gesamtzahl der Substituenten naturgemäß durch die maximale Anzahl der zu substituierenden Positionen am aromatischen Grundkörper begrenzt ist. Als Alkylsubstituenten seien beispielsweise solche mit 1 bis 4, bevorzugt 1 bis 2, bevorzugt 1 C-Atom genannt, wie Methyl, Ethyl, Propyl, Isopropyl, Butyl oder Isobutyl.

Beispiele für solche Lösungsmittel sind Toluol, Xylol, Trimethylbenzol, Ethylbenzol, Methylnaphthalin, Tetrahydronaphthalin, Methylanthracen, Methyl-Diphenyl, Chlorbenzol, Brombenzol, 1,2-Dichlorbenzol, 1,3-Dichlorbenzol, 1,4-Dichlorbenzol, 1,2-Dibrombenzol, 1,3-Dibrombenzol, 1,4-Dibrombenzol, 1,2,4-Trichlorbenzol 1,2,3-Trichlorbenzol, 1,3,5-Trichlorbenzol, 2-Chlor-toluol, 3-Chlor-toluol, 4-Chlor-toluol, 2-Brom-toluol, 3-Brom-toluol, 4-Brom-toluol, 2,6-Dichlortoluol, 2,4-Dichlortoluol, 2,5-Dichlortoluol, 2,3-Dichlortoluol, 3,4-Dichlortoluol und Sulfone wie Tetramethylensulfon, Dimethylsulfon oder Diethylsulfon. Solche Lösungsmittel können sowohl einzeln als auch im Gemisch angewendet werden, beispielsweise technische Dichlorbenzol-, Dichlortoluol- oder Trichlorbenzolgemische. Als Lösungsmittel können aber auch aliphatische Kohlenwasserstoffe, wie Petroleum, Kerosin oder Paraffine, wie Isododecan oder Decalin, sowie deren Gemische mit geeigneten Siedepunkten eingesetzt werden.

Das Lösungsmittel wird in einer Menge von 100 bis 2000 ml, bevorzugt 100 bis 500 ml und besonders bevorzugt 150 bis 250 ml, pro Mol Anilin eingesetzt.

Als Lösungsmittel werden bevorzugt alkyl- und/oder halogensubstituierte Benzole eingesetzt, bei denen selbstverständlich die Gesamtzahl der Substituenten auf maximal 6 begrenzt ist. Besonders bevorzugt werden durch 1 bis 3 Halogenatome substituierte Benzole eingesetzt, die daneben noch eine Methylgruppe tragen können. In ganz besonders bevorzugter Weise werden Dichlor- und/oder Trichlorbenzole und/oder -toluole eingesetzt.

Weiterhin können Lösungsmittel der obengenannten Art, die bereits im erfindungsgemäßen Verfahren eingesetzt waren und die nach der Abtrennung des Reaktionsproduktes p-Sulfanilsäure in freier oder salzartiger Form gegebenenfalls Anilin enthalten, ohne weitere Reinigung dem erfindungsgemäßen Verfahren wieder zugeführt werden.

Als Schwefelsäure wird zweckmäßigerweise das Monohydrat (=100%ige $H_2SO_4$) eingesetzt. Selbstverständlich sind auch Schwefelsäuren mit verschiedenen Wassergehalten geeignet. Je höher der Wassergehalt der eingesetzten Schwefelsäure ist, desto länger muß jedoch die Reaktionszeit angesetzt werden, da neben dem Reaktionswasser auch als Verdünnungswasser aus der Schwefelsäure aus dem Reaktionsgemisch entfernt werden muß.

Die Reihenfolge des Zusammengebens der Reaktionspartner im erfindungsgemäßen Verfahren ist beliebig. Vorzugsweise wird das Anilin im Lösungsmittel vorgelegt und die Schwefelsäure zugetropft. Ebenso ist jedoch ein Vorlegen der Schwefelsäure im Lösungsmittel und eine darauf folgende Zugabe des Anilins möglich. In einer weiteren Variante schließlich kann ein gesondert hergestelltes gegebenenfalls wasserhaltiges Aniliniumsulfat als Schmelze oder als Aufschlämmung in einem der genannten Lösungsmittel in vorgelegtes Lösungsmittel gegeben werden. Das erfindungsgemäße Verfahren kann neben der chargenweisen Durchführung auch kontinuierlich durchgeführt werden, wobei Anilin, Schwefelsäure und das Lösungsmittel gleichzeitig zugegeben werden. In allen beschriebenen Fällen ist die Einhaltung des oben beschriebenen Mindestüberschusses an Anilin erforderlich.

Erfindungsgemäß werden die Schwefelsäure und das überschüssige Anilin in einem der beschriebenen Lösungsmittel oder Lösungsmittelgemische so umgesetzt, daß die Entfernung des Reaktionswassers aus dem Reaktionsgemisch mindestens teilweise bei 180 bis 280° C, bevorzugt 190 bis 260° C, besonders bevorzugt 200 bis 250° C, durchgeführt wird.

Als mindestens teilweise Entfernung des Reaktionswassers im erfindungsgemäßen Temperaturbereich sei beispielsweise eine Menge von 10 bis 100%, bevorzugt 20 bis 100%, besonders bevorzugt 30 bis 100%, des gesamten aus den Einsatzmengen zu erwartenden Reaktionswassers genannt. Für den Fall, daß weniger als 100% des Reaktionswassers im erfindungsgemäßen Temperaturbereich aus dem Reaktionsgemisch herausdestilliert werden, kann die Differenz zu 100% an Reaktionswasser, beispielsweise 90% oder weniger, bevorzugt 80% oder weniger, besonders bevorzugt 70% oder weniger, auch unterhalb von 180° C, beispielsweise beim Aufheizen des Reaktionsgemisches aus dem Reaktionsgemisch herausdestilliert werden.

Die Entfernung des Reaktionswassers kann beisielsweise durch Azeotropdestillation mit dem Lösungsmittel erfolgen. Bei hochsiedenden Lösungsmitteln wird das Reaktionswasser beispielsweise durch einfaches Abdestillieren aus dem Reaktionsgemisch entfernt. Die Wasserentfernung kann stetig erfolgen, beispielsweise bei der azeotropen Destillation unter atmosphärischem Druck, sie kann aber auch diskontinuierlich vorgenommen werden, beispielsweise durch absatzweises Abdestillieren des Reaktionswassers aus dem geschlossenen Druckreaktor.

Das erfindungsgemäße Verfahren kann bei Normaldruck, bei Unterdruck oder bei Überdruck durchgeführt werden. Die Arbeitsweise bei Überdruck ist beispielsweise dann erforderlich, wenn ein Lösungsmittel verwendet wird, dessen Siedepunkt unterhalb des Temperaturbereiches von 180 bis 280° C liegt. Die Anwendung von Überdruck kommt weiterhin dann in Frage, wenn ein Lösungsmittel zwar im Bereich der genannten Reaktionstemperatur siedet, aber durch die im erfindungsgemäßen Verfahren erfolgende Azeotropdestillation des Wassers mit diesem Lösungsmittel die gewünschte Reaktionstemperatur ohne Überdruck nicht erreicht werden kann. Bei der Arbeitsweise mit Überdruck sind die Lösungsmittelmengen, die bis zur vollständigen Entfernung des Reaktionswassers gleichzeitig abdestillieren, besonders gering. Die Anwendung von Unterdruck kommt beispielsweise dann in Frage, wenn eine sehr hochsiedende inerte Verbindung als Lösungsmittel eingesetzt wird und unter Rückflußbedingungen gearbeitet werden soll, wenn also die gewünschten Rückflußbedingungen innerhalb des Temperaturbereiches von 180 bis 280° C nur durch Unterdruck zu erreichen sind.

Aus den aufgezeigten Zusammenhängen wird ersichtlich, daß der Druck für das erfindungsgemäße Verfahren innerhalb eines weiten Bereiches eingestellt werden kann, beispielsweise von 0,1 bis 100 bar, bevorzugt 1 bis 50 bar, besonders bevorzugt 1 bis 20 bar. Wird unter Überdruck gearbeitet, so wird ein Druck von 1,1 bis 10 bar eingestellt. In ganz besonders bevorzugter Weise wird unter dem Eigendruck des Reaktionsgemisches bei der Reaktionstemperatur gearbeitet, der sich aus dem Partialdruck des Reaktionswassers und dem des Lösungsmittels zusammensetzt.

Beispielhaft für die Ausführung des erfindungsgemäßen Verfahrens seien die folgenden Varianten genannt:

a) Bei der drucklosen Ausführungsform wird Anilin im Lösungsmittel vorgelegt und Monohydrat zugetropft. Der gesamte Reaktionsansatz wird anschließend bei atmosphärischem Druck auf eine Temperatur von 180 bis 280° C erhitzt. Nach Erreichen dieses Temperaturbereiches wird das Reaktionswasser, gegebenenfalls gemeinsam mit etwas Lösungsmittel, abdestilliert. Das Lösungsmittel kann nach dem Abtrennen des mitdestillierten Reaktionswassers in den Ansatz zurückgeführt werden. Diese Variante kann auch so durchgeführt werden, daß ein Teil des Reaktionswassers, beispielsweise bis zu 90%, bevorzugt bis zu 80% und besonders bevorzugt bis zu 70%, auch bereits bei einer Temperatur von unter 180° C, gegebenenfalls unter Anwendung von Unterdruck, aus dem Reaktionsansatz herausdestilliert wird.

Beispielsweise wird Anilin in einem 0,5–2%igen, bevorzugt etwa 1%igen Überschuß in 1,2,4-Trichlorbenzol vorgelegt und Monohydrat ohne Kühlung zugetropft. Der Reaktionsansatz wird bei atmosphärischem Druck zum Sieden erhitzt. Das gesamte Reaktionswasser wird im Temperaturbereich von 190 bis 210° C durch Destillation gemeinsam mit etwas 1,2,4-Trichlorbenzol entfernt. Nach Abtrennung des Wassers in einem Wasserabscheider wird das Lösungsmittel in den Ansatz zurückgegeben.

b) Bei der Ausführungsform unter Überdruck wird das aus Anilin, Lösungsmittel und Monohydrat

3

bestehende Reaktionsgemisch in einem geschlossenen Reaktionsgfäß auf eine Temperatur von 180 bis 280°C gebracht. Das entstehende Reaktionswasser, wird gegebenenfalls gemeinsam mit etwas Lösungmsittel, enntweder stetig oder absatzweise aus dem Reaktionsgemisch entfernt. Bei der absatzweisen Durchführung der Wasserentfernung kann es, besonders bei der Verwendung von niedrig siedenden Lösungsmitteln, vorteilhaft sein, vor der absatzweisen Wasser/Lösungsmittel-Destillation das Erreichen eines höheren Druckes zuzulassen, weil dann die Mengen an mitdestilliertem Lösungsmittel besonders gering gehalten werden können. Auch bei der Ausführungsform unter Überdruck kann das mit dem Reaktionswasser abdestillierte Lösungsmittel wieder in den Reaktionsansatz zurückgeführt werden. Hierzu wird das Destillat, wie bereits beschrieben, beispielsweise in einen Wasserabscheider oder eine andere geeignete Apparatur geleitet, aus der das vom Wasser befreite Lösungsmittel entnommen und in das Reaktionsgefäß zurückgeführt wird. An den in der Trennapparatur zurückbleibenden Wassermengen kann jeweils der Fortschritt und das Ende der Reaktion des erfindungsgemäßen Verfahrens erkannt werden. Der Vorteil der Ausführungsvariante bei Überdruck liegt darin, daß eine p-Sulfanilsäure mit einem sehr geringen Gehalt an Anilin-2,4-disulfonsäure gewonnen wird und daß auch Lösungsmittel mit Siedepunkten von unter 180°C eingesetzt werden können.

Um das Reaktormaterial und die Dichtungen der Reaktoranschlüsse den erforderlichen hohen Temperaturen möglichst kurzzeitig auszusetzen, kann diese Ausführungsvariante auch so abgewandelt werden, daß aus dem Reaktionsansatz eine Menge von bis zu 90% des Reaktionswassers bei einer Temperatur unterhalb von 180°C bei Normaldruck herausdestilliert wird. Anschließend wird der Reaktor verschlossen und auf eine Temperatur von 180 bis 280°C erhitzt, gegebenenfalls die Einstellung der gewünschten Temperatur unter Durchmischen des Reaktionsansatzes abgewartet und dann die verbleibende Menge von mindestens 10% des Reaktionswassers aus dem Ansatz kontinuierlich oder absatzweise herausdestilliert.

Beispielsweise werden Anilin in einem 0,5—2%igen, bevorzugt etwa 1%igen Überschuß und 1,2-Dichlorbenzol oder ein Gemisch der isomeren Dichlorbenzole in einem verschließbaren Reaktor vorgelegt und das Monohydrat gegebenenfalls unter Kühlung zugegeben. Danach wird der geschlossene Reaktor auf eine Innentemperatur von etwa 200°C bis 210°C erhitzt, wobei sich der zugehörige Überdruck aufbaut. In der oben beschriebenen Weise wird nunmehr das gesamte Reaktionswasser bei dieser Temperatur absatzweise so entnommen, daß möglichst wenig Lösungsmittel mitgeschleppt wird.

Die genannten Ausführungsformen der erfindungsgemäßen Entfernung von mindestens einem Teil des Reaktionswassers bei einer Temperatur von 180 bis 280°C können selbstverständlich auch auf eine kontinuierliche Verfahrensdurchführung angewandt werden, wobei ebenfalls die Wasserabnahme aus dem Reaktionsansatz zu höchstens 90% bei einer Temperatur unterhalb 180°C und zu mindestens 10% im Temperaturbereich von 180 bis 280°C erfolgt.

Das nach dem vollständigen Abdestillieren des Reaktionswassers verbleibende Reaktionsgemisch kann, gegebenenfalls nach Abkühlung auf etwa 100°C, in verschiedener Weise aufgearbeitet werden. So ist es beispielsweise möglich, die im Lösungsmittel nicht oder nur wenig lösliche p-Sulfanilsäure durch Abfiltrieren oder Zentrifugieren vom Lösungsmittel zu trennen. Die p-Sulfanilsäure liegt dann nach dem Trocknen in freier Form vor. Das hierbei etwa als Filtrat, zurückgewonnene Lösungsmittel kann in dieser Form ohne weitere Reinigung für einen nächsten Ansatz verwendet werden.

. Zur Gewinnung der p-Sulfanilsäure kann das Reaktionsgemisch nach Abkühlung auch einer alkalisch-wäßrigen Extraktion unterzogen werden, wobei die p-Sulfanilsäure in Form ihres Salzes in die wäßrige Phase übergeht. Als alkalisch reagierende Stoffe für eine solche alkalisch-wäßrige Extraktion können beispielsweise Natriumhydroxid, Kaliumhydroxid, Calciumhydroxid, Magnesiumhydroxid, Ammoniak oder aliphatische Amine eingesetzt werden. Das überschüssige Anilin verbleibt hierbei in der organischen Lösungsmittelphase. Diese organische Phase kann in üblicher Weise von dem wäßrigen Extrakt abgetrennt werden und in dieser Form wieder in einem Folgeansatz ganz oder teilweise eingesetzt werden. Der wäßrige Extrakt wird sodann zweckmäßigerweise durch Andestillieren von Spuren des Lösungsmittels befreit. Man erhält so eine klare, nahezu farblose Salzlösung der p-Sulfanilsäure, die ohne jegliche weitere Reinigung, beispielsweise für die Darstellung von optischen Aufhellern oder für andere Zwecke, eingesetzt werden kann. Infolge der erfindungsgemäßen Anwendung eines Anilinüberschusses kann somit auf eine Filtration von unlöslichen Rückständen und eine Reinigung beisielsweise mit A-Kohle verzichtet werden, zwei in der Literatur als notwendig beschriebene Reinigungsoperationen.

Dieser wäßrige Extrakt kann jedoch auch durch Eindampfen auf das der verwendeten alkalischen Verbindung korrespondierende Salz der p-Sulfanilsäure aufgearbeitet werden. Weiterhin kann dieser Extrakt auch durch Ansäuern mit einer Mineralsäure, wie Salzsäure oder Schwefelsäure behandelt werden, wobei die freie, sehr reine p-Sulfanilsäure ausfällt und beispielsweise durch Filtration gewonnen werden kann. Zum Ansäurern sind hierbei mindestens äquivalente Mengen Mineralsäure, bezogen auf die eingesetzte alkalische Verbindung, erforderlich, um eine größtmögliche Ausbeute an p-Sulfan-ilsäure zu erzielen.

Mit Hilfe des erfindungsgemäßen Verfahrens kann direkt eine sehr reine p-Sulfanilsäure hergestellt werden, deren Gehalt an Anilin-2,4-disulfonsäure kleiner als 4 Gew.-% bevorzugt kleiner als 1 Gew.-%, besonders bevorzugt kleiner als 0,7 Gew.-%, bezogen auf die Menge an p-Sulfanilsäure, ist. Die erfindungsgemäß herstellbare p-Sulfanilsäure stellt zusätzlich ein äußerst helles Produkt dar, beispielsweise gemessen an der Farbzahl nach Hazen einer 20%igen wäßrigen Natrium-Sulfanilat-Lösung. Diese Hazen-Farbzahl ist bei einer erfindungsgemäß hergestellten p-Sulfanilsäure 2, während sie bei nachgearbeiteten Beispielen aus der oben angegebenen Literatur bei Verwendung von äquimolaren Mengen Anilin oder bei Anwendung eines geringen Schwefelsäureüberschusses den Hazen-Wert von 13 annimmt. Diese Tatsache ist beispielsweise für den Einsatz der p-Sulfanilsäure zur Darstellung von optischen Aufhellern von großer Bedeutung.

Weiterhin entfällt das in der Literatur beschriebene und bei der Nachbearbeitung bestätigte Abfiltrieren von dunklen unlöslichen Rückständen.

Ein weiterer Vorteil des erfindungsgemäßen Verfahrens besteht in den überraschend kurzen Reaktionszeiten. Zudem braucht bei der Arbeitsweise unter erhöhtem Druck auch beim Einsatz von tiefer siedenden Lösungsmitteln nur eine kleine Menge Lösungsmittel abdestilliert zu werden, um das Reaktionswasser vollständig zu entfernen. Dadurch können die Energiekosten des Verfahrens niedrig gehalten werden. Die überraschend festgestellte hohe Reinheit des Reaktionsgemisches wirkt sich nicht nur günstig auf die beschriebene Qualität der p-Sulfanilsäure aus, sondern ermöglicht auch die erfindungsgemäße Wiederverwendung des organischen Lösungsmittels und des gegebenenfalls darin enthaltenen Anilins, sowie die einfache Aufarbeitung des Reaktionsgemisches, beispielsweise durch bloße Filtration oder durch Phasentrennung bei wäßriger Extraktion, wodurch die ebenfalls energieaufwendige Destillation des Lösungsmittels entfällt.

## Beispiele

### Beispiel 1 (zum Vergleich)

(nach B. I. Kissin, E. N. Kurakin, Khim. Prom. 41, 104 (1965))

186,2 g (2 Mol) Anilin werden in 500 ml 1,2-Dichlorbenzol vorgelegt und 208,25 g (2,04 Mol) 96%ige Schwefelsäure zugetropft. Es wird 8 Stunden am Wasserabscheider auf Siedetemperatur erhitzt, abgekühlt und abgesaugt. Der Niederschlag wiegt nach dem Trocknen 338,5 g.

Der Niederschlag enthält 86,1% p-Sulfanilsäure (84,2% Ausbeute, bezogen auf Anilin) und 11,2% Anilin-2,4-disulfonsäure (7,5%, bezogen auf Anilin).

### Beispiel 2

470,1 g (5,05 Mol) Anilin werden in 1000 ml o-Dichlorbenzol in einem Rührautoklaven vorgelegt und 490 g (5,0 Mol) Monohydrat in 30 Minuten ohne Kühlung zugetropft, wobei die Temperatur auf 120°C ansteigt. Dann wird aufgeheizt und bei 210°C Innentemperatur das erste Reaktionswasser mit etwas o-Dichlorbenzol abdestilliert. Es wird im Verlaufe von 1,5 Stunden auf diese Weise das Reaktionswasser aus dem Autoklaven entnommen, mitgerissene o-Dichlorbenzol wird durch frisches ersetzt. Die entstandene p-Sulfanilsäure wird mit wäßriger Kaliumhydroxidlösung extrahiert, die beiden entstandenen Phasen werden getrennt und die wäßrige Phase zur Trockene eingeengt. Der Rückstand wiegt nach dem Trocknen 1041 g und enthält 78,8 Gew.-% p-Sulfanilsäure und 0,5 Gew.-% Anilin-2,4-disulfonsäure (jeweils gerechnet als freie Säure). Die Ausbeute an p-Sulfanilsäure beträgt 94,6% der theoretischen Ausbeute.

### Beispiel 3 (zum Vergleich)

(nach B. I. Kissin, UdSSR-Patent Nr. 667 550 (30. 5. 79))

150 g (1,61 Mol) Anilin werden in einem Gemisch von 310 ml 1,2-Dichlorbenzol und 62 ml 1,2,4-Trichlorbenzol vorgelegt. Innerhalb von 60 Minuten werden 160 g (1,63 Mol) Monohydrat zugetropft, wobei die Temperatur auf 123°C ansteigt. Es wird zum Sieden (172°C) erhitzt und im Laufe von 8 Stunden Wasser (28 ml) und Lösungsmittelgemisch (186 ml) abdestilliert, wobei die Temperatur bis auf 182°C ansteigt, und anschließend weitere 6 Stunden am Wasserabscheider erhitzt. Man erhält eine dunkle, dickflüssige Suspension. Im Wasserstrahlvakuum wird restliches Lösungsmittel abdestilliert (154 ml), der Rückstand mit 450 ml Wasser versetzt und mit 120 ml 40%iger NaOH neutralisiert. Die Lösung wird von schwarzen Rückständen abgesaugt, anschließend 1,0 Stunden mit Wasserdampf destilliert und zur Trockene eingeengt. Der graue Rückstand wiegt nach dem Trocknen 295 g und enthält 84,9 Gew.-% p-Sulfanilsäure (89,8% Ausbeute, bezogen auf Anilin bzw. 88,7%, bezogen auf $H_2SO_4$) und 2,2 Gew.-% Anilin-2,4-disulfonsäure). Farbzahl nach Hazen (20%ige wäßrige Natriumsalz-

Lösung) = 13.

### Beispiel 4

235,2 g (2,525 Mol) Anilin werden bei 85°C in 500 ml 1,2,4-Trichlorbenzol vorgelegt und 245,3 g (2,5 Mol) Monohydrat in 15 Minuten ohne Kühlung zugetropft, wobei die Temperatur auf 155 bis 160°C ansteigt. Es wird 1,5 Stunden am Wasserabscheider erhitzt, wobei die Innentemperatur von 190 auf 211°C ansteigt. Es wird auf 100°C abgekühlt und die weiße Suspension mit 870 ml Wasser und 281,6 g (2,51 Mol) 50%iger wäßriger Kaliumhydroxidlösung versetzt. In der Hitze werden die Phasen getrennt. Die organische Phase enthält 0,7% Anilin und kann für weitere Ansätze ohne weitere Reinigung wiederverwendet werden.

Die wäßrige Phase wird andestilliert, um Spuren von Lösungsmittel zu entfernen. Sie wiegt 1168 g und enthält 34,9% p-Sulfanilsäure, gerechnet als freie p-Sulfanilsäure (95,6% Ausbeute, bezogen auf umgesetztes Anilin) und 1,1 Gew.-% Anilin-2,4-disulfonsäure.

### Beispiel 5

470,1 g (5,05 Mol) Anilin werden in 1000 ml 1,2,4-Trichlorbenzol in einem Rührautoklaven vorgelegt und 490 g (5,0 Mol) Monohydrat in 30 Minuten zugetropft. Man heizt den geschlossenen Autoklaven auf 210°C Innentemperatur und entfernt das Reaktionswasser mit geringen Lösungsmittelmengen durch Entspannen über einen Kühler. Nach 60 Minuten ist das gesamte Reaktionswasser (90 ml), zusammen mit 74 ml Lösungsmittel, übergegangen. Man rührt noch 30 Minuten nach.

Die entstandene p-Sulfanilsäure wird, wie in Beispiel 2 beschrieben, als Kaliumsalz isoliert (1065,1 g; 96%ig). Ausbeute an p-Sulfanilsäure 96,7%; Gehalt an Anilin-2,4-disulfonsäure 0,7%

### Beispiele 6 und 7

### Sulfonierung wie in Beispiel 4 beschrieben

Die Suspensionen werden mit Wasser und mit einer der in der Tabelle angegebenen wäßrigen Basen versetzt. Nach Phasentrennung werden die wäßrigen Phasen mit 100%iger $H_2SO_4$ (Monohydrat) angesäuert und der entstandene Niederschlag abgesaugt. Der Niederschlag enthält neben der p-Sulfanilsäure etwas Anilin-2,4-disulfosäure und etwas mitgerissenes durch Neutralisation entstandenes anorganisches Salz.

| Nr. Base | Menge (Mol) | $H_2SO_4$ (Mol) | $H_2O$-Vol. (ml) | Gehalte (Gew.-%) an | | Ausb. an Sulf. bez. auf umgesetztes | |
|---|---|---|---|---|---|---|---|
| | | | | Disulfosre. | Sulf.sre | Anilin (%) | $H_2SO_4$ (%) |
| 6 KOH | 2,51 | 2,5 | 370 | 0,68 | 88,2 | 95,1 | 93,6 |
| 7 $NH_3$ | 2,51 | 1,25 | 290 | 0,65 | 96,8 | 95,9 | 94,4 |

### Beispiel 8

235,2 g (2,525 Mol) Anilin werden bei 85°C in 500 ml 1,2,4-Trichlorbenzol vorgelegt und 245,3 g (2,5 Mol) Monohydrat in 15 Minuten ohne Kühlung zugetropft, wobei die Temperatur auf 155 bis 160°C ansteigt. Es wird nun bis zum Sieden erhitzt und innerhalb von 30 Minuten 247 ml Lösungsmittel und 32 ml Wasser abdestilliert (Temperatur 189 bis 197°C). Es wird noch 1 Stunde am Wasserabscheider bei 200 bis 210°C erhitzt, wobei sich noch 8 ml Wasser abscheiden lassen. Im Wasserstrahlvakuum wird zur Trockene eingeengt und der weiße Rückstand mit 700 ml Wasser und 176 ml 40%iger Natronlauge neutralisiert.

Die kaum gefärbte Lösung wird zur Trockene eingeengt. Das Gewicht des trockenen, hellen Rückstandes beträgt 481,0 g und enthält 85,6% p-Sulfanilsäure (96,5%ig, Umrechnung des Na-Salzes auf freie Säure). Dies entspricht einer Ausbeute von 96,2% (bezogen auf umgesetztes Anilin). Farbzahl nach Hazen (20%ige wäßrige Natriumsalz-Lösung) = 2.

**0 063 270**

Beispiel 9

(zum Vergleich nach Russ- Patent Nr. 667 550)

150 g (1,61 Mol) Anilin werden in 310 ml 1,2,4-Trichlorbenzol vorgelegt und 160 g (1,63 Mol) Monohydrat zugetropft, wobei die Temperatur auf 116°C ansteigt. Innerhalb 50 Minuten werden 169 ml Lösungsmittel und 23 ml Wasser abdestilliert, hierbei steigt die Kolbeninnentemperatur bis 198°C an. Es wird noch 1 Stunde am Wasserabscheider erhitzt (Endtemperatur im Kolben: 210°C), wobei sich noch 4 ml Wasser abscheiden.

Die grau gefärbte Suspension wird im Wasserstrahlvakuum bis zur Trockene eingeengt. Der Rückstand wird mit 450 ml Wasser versetzt und mit 112 ml 40%iger NaOH neutralisiert. Die dunkelgefärbte Lösung wird von unlöslichen Rückständen abfiltriert und zur Trockene eingeengt. Das Gewicht des braunen Rückstandes beträgt 329 g. Er enthält 79,2 Gew.-% p-Sulfanilsäure (89,3%ig, berechnet als Natriumsalz). Dies entspricht einer Ausbeute von 93,4% (bezogen auf Anilin). Farbzahl nach Hazen (20%ige wäßrige Natriumsalz-Lösung) = 13.

Beispiel 10

500 ml 1,2,4-Trichlorbenzol werden vorgelegt und auf 90°C erwärmt. Dann werden zunächst von 232,8 g = 2,50 Mol Anilin 5 ml Anilin zugegeben und der Rest gleichzeitig mit 242,8 g = 2,47 Mol Schwefelsäure zudosiert.

Hierfür werden 30 Minuten benötigt, und die Temperatur steigt auf 152°C an. Man erhitzt während 1,5 Stunden am Wasserabscheider zum Sieden, wobei die Innentemperatur von 190°C bis 211°C ansteigt.

Die weiße Suspension wird abgesaugt und getrocknet. Die 422 g Feststoff enthalten 95 Gew.-% p-Sulfanilsäure, was einer Ausbeute von 94,6% entspricht.

Beispiel 11

In einem Dreihalskolben mit Bodenablaß wird aus 47,0 g = 0,505 Mol Anilin und 49,0 g = 0,50 Mol Monohydrat in 500 ml 1,2,4-Trichlorbenzol eine Suspension von Aniliniumsulfat hergestellt. Diese Suspension wird so in vorgelegtes siedendes Trichlorbenzol (1000 ml) dosiert, daß die Innentemperatur 200°C nicht unterschreitet. Aus dem 2. Reaktionsgefäß werden Lösungsmittel und Reaktionswasser abdestilliert. Nach beendeter Zugabe der Suspension wird 30 Minuten nachgerührt, die weiße Suspension abgesaugt und getrocknet. Die Ausbeute beträgt 95,4%, der Gehalt an Anilin-2,4-disulfosäure beträgt 2,6%.

Beispiel 12

Ein Reaktionsansatz wie in Beispiel 5 wird auf 200°C Innentemperatur erhitzt. Es werden 90 ml Reaktionswasser zusammen mit 65 ml Lösungsmittel aus dem Ansatz entfernt. Der Reaktionsansatz wird wie in Beispiel 2 aufgearbeitet. Die Ausbeute an p-Sulfanilsäure beträgt 96,5% der theoretischen Ausbeute, der Gehalt an Anilin-2,4-disulfonsäure beträgt 0,9 Gew.-%

Beispiel 13

470,1 g (5,05 Mol) Anilin werden in 1000 ml 1,2,4-Trichlorbenzol in einem Rührautoklaven vorgelegt und 490 g (5,0 Mol) Monohydrat in 15 Minuten zugetropft. Man heizt den geschlossenen Autoklaven auf 240°C Innentemperatur und entfernt das Reaktionswasser (90 ml) mit geringen Lösungsmittelmengen (58 ml) durch Entspannen und Kondensieren im Verlaufe von 40 Minuten. Man rührt noch 50 Minuten bei dieser Temepratur nach. Dann werden 3100 ml Wasser und 374 ml 50%ige Kaliumhydroxidlösung zugegeben, die Phasen getrennt und von der wäßrigen Phase 460 ml abdestilliert. Die wäßrige Kaliumsalzlösung wiegt sodann 4218,5 g und enthält 20,1 Gew.-% p-Sulfanilsäure (98% der theoretischen Ausbeute). Der Gehalt an Anilin-2,4-disulfonsäure beträgt 0,054 Gew.-%.

7

## Beispiel 14

Der Reaktionsansatz wie im Beispiel 5 wird nach der Schwefelsäurezugabe auf 250°C Innentemperatur erhitzt. Es werden 90 ml Reaktionswasser und 45 ml Lösungsmittel aus dem Ansatz entfernt. Die Aufarbeitung erfolgt wie im Beispiel 2. Die Ausbeute an p-Sulfanilsäure beträgt 93,6% der theoretischen Ausbeute, der Gehalt an Anilin-2,4-disulfonsäure beträgt 0,13 Gew.-%.

## Beispiel 15

In einem Rührautoklaven werden 1000 ml 1,2,4-Trichlorbenzol bei 210°C vorgelegt und eine Schmelze aus 470,1 g (5,05 Mol) Anilin und 490 g (5,0 Mol) Monohydrat so eingepumpt, daß während des Zupumpens die Reaktionstemperatur von 210°C gehalten wird. Gleichzeitig wird das entstehende Reaktionswasser mit geringen Lösungsmittelmengen abdestilliert. Das Zupumpen der Schmelze dauert 1,5 Stunden, die Nachrührzeit bei 210°C 30 Minuten. Die körnige Suspension wird abgesaugt und getrocknet. Man erhält 850,7 g Feststoff mit 96,3 Gew.-% p-Sulfanilsäure (94,6% der theoretischen Ausbeute).

## Beispiel 16

470,1 g (5,05 Mol) Anilin werden in 1000 ml Dichlortoluol in einem Rührautoklaven vorgelegt und 490 g (5,0 Mol) Monohydrat in 30 Minuten zugetropft. Die Temperatur steigt auf etwa 150°C an. Es wird auf 210°C geheizt und bei dieser Temperatur das Reaktionswasser mit einem geringen Anteil Lösungsmittel entnommen. Nach etwa 60 Minuten ist die Entfernung des Reaktionswassers beendet, und es wird noch 30 Minuten nachgerührt. Der Reaktionsansatz wird wie in Beispiel 2 aufgearbeitet. Man erhält 1014,2 g Kaliumsalz der p-Sulfanilsäure (98,4%ig). der Gehalt an freier p-Sulfanilsäure beträgt 80,7% (94,5% der theoretischen Ausbeute), der Gehalt an Anilin-2,4-disulfonsäure beträgt 1,0%.

## Beispiel 17

Ansatz und Reaktionsdurchführung wie in Beispiel 16, jedoch wird als Lösungsmittel Chlorbenzol benutzt. Die Reaktionszeit beträgt 1 Stunde/50 Minuten. Nach der Aufarbeitung erhält man 1015,2 g Kaliumsalz der p-Sulfanilsäure (97,0%ig). Der Gehalt an freier p-Sulfanilsäure beträgt 79,5% (93,2% der theoretischen Ausbeute), der Gehalt an Anilin-2,4-disulfonsäure beträgt 0,7%

## Beispiel 18

470,1 g (5,05 Mol) Anilin werden in 1000 ml o-Dichlorbenzol in einem Rührautoklaven vorgelegt und 490 g (5,0 Mol) Monohydrat in 10 Minuten zugetropft. Dann werden bei 175 bis 180°C Innentemperatur 72 ml Wasser abdestilliert. Der Reaktor wird danach verschlossen und auf 210°C Innentemperatur geheizt. Es wird 15 Minuten gerührt und danach werden restliche 18 ml Reaktionswasser abdestilliert. Die Feststoffe des Reaktionsansatzes werden abgesaugt und getrocknet; man erhält dabei 832,6 g. Die Ausbeute an p-Sulfanilsäure beträgt 93,3% der theoretischen Ausbeute, der Gehalt an Anilin-2,4-Disulfonsäure beträgt 2,5 Gew.-%.

## Beispiel 19

470,1 g (5,05 Mol) Anilin werden in 1000 ml Tetralin in einem Rührautoklaven vorgelegt und 490 g (5,0 Mol) Monohydrat in 15 Minuten angetropft. Man heizt den geschlossenen Autoklaven auf 210°C Innentemperaur und entfernt das Reaktionswasser (90 ml) mit geringen Lösungsmittelmengen (60 ml) durch Entspannen und Kondensieren im Verlaufe von 60 Minuten. Man rührt noch 10 Minuten bei dieser Temepratur nach. Die Feststoffe des Reaktionsansatzes werden abgesaugt und getrocknet; man erhält 864,2 g. Die Ausbeute an p-Sulfanilsäure beträgt 97,8% der theoretischen Ausbeute; der Gehalt an Anilin-2,4-disulfonsäure beträgt 0,37 Gew.-%

**Patentansprüche**

1. Verfahren zur Herstellung von p-Sulfanilsäure nach dem sogenannten Backverfahren durch Umsetzung von Anilinhydrogensulfat bei erhöhter Temperatur in einem inerten Lösungsmittel, dadurch gekennzeichnet, daß die Umsetzung in Gegenwart eines Überschusses von Anilin, der über das äquimolare Verhältnis von Anilin und Schwefelsäure hinausgeht, vorgenommen wird und daß die Entfernung des Reaktionswasser aus dem Reaktionsgemisch mindestens teilweise bei 180 bis 280° C, gegebenenfalls unter Druck, durchgeführt wird.

2. Verfahren nach Anspruch 1, dadurch gekennzeichnet, daß Anilin in einem Überschuß von mindestens 0,01 Mol-% eingesetzt wird.

3. Verfahren nach Anspruch 1 und 2, dadurch gekennzeichnet, daß der Anilinüberschuß 0,05 bis 10 Mol-% beträgt.

4. Verfahren nach Anspruch 1 bis 3, dadurch gekennzeichnet, daß die mindestens teilweise Entfernung des Reaktionswassers bei 190 bis 260° C durchgeführt wird.

5. Verfahren nach Anspruch 1 bis 4, dadurch gekennzeichnet, daß die mindestens teilweise Entfernung des Reaktionswassers 10 bis 100% des gesamten Reaktionswassers umfaßt.

6. Verfahren nach Anspruch 1 bis 4, dadurch gekennzeichnet, daß die mindestens teilweise Entfernung des Reaktionswassers 20 bis 100% des gesamten Reaktionswassers umfaßt.

7. Verfahren nach Anspruch 1 bis 6, dadurch gekennzeichnet, daß Anilin in einem molaren Überschuß von 0,5—2% und Schwefelsäure in Trichlorbenzol bei atmosphärischem Druck so erhitzt werden, daß das gesamte Reaktionswasser bei etwa 210° C entfernt wird.

8. Verfahren nach Anspruch 1 bis 6, dadurch gekennzeichnet, daß Anilin in einem molaren Überschuß von 0,5—2% und Schwefelsäure in einem geschlossenen Reaktor in Dichlorbenzol unter Eigendruck auf etwa 200° C bis 210° C erhitzt werden und das gesamte Reaktionswasser bei dieser Temperatur entfernt wird.

9. Verfahren nach Anspruch 1 bis 8, dadurch gekennzeichnet, daß die p-Sulfanilsäure in freier Form durch Abfiltrieren aus dem Reaktionsgemisch und anschließendes Trocknen erhalten wird und das dabei entstehende Filtrat in die Reaktion zurückgeführt wird.

10. Verfahren nach Anspruch 1 bis 8, dadurch gekennzeichnet, daß die p-Sulfanilsäure in Salzform durch alkalisch-wäßrige Extraktion aus dem Reaktionsgemisch erhalten wird und die verbleibende organische Phase in die Reaktion zurückgeführt wird, wobei der wäßrig-alkalische Extrakt

a) als solcher und damit als wäßrige Lösung des p-Sulfanilsäure-Salzes weiterverarbeitet wird oder
b) eingedampft wird und das p-Sulfanilsäure-Salz gewonnen wird oder
c) angesäuert wird und die dabei ausgefallene freie p-Sulfanilsäure durch Absaugen des Niederschlages gewonnen wird.

## Claims

1. Process for the preparation of p-sulphanilic acid by the so-called baking process by reacting anilinium hydrogen sulphate at an elevated temeprature in an inert solvent, characterised in that the reaction is carried out in the presence of an excess of aniline which is greater than the equimolar ratio of aniline and sulphurio acid, and in that at least some of the reaction water is removed from the reaction mixture at 180 to 280° C, if appropriate under pressure.

2. Process according to Claim 1, characterised in that aniline is udes in an excess fo at least 0.01 mol %.

3. Process according to Claims 1 and 2, characterised in that the aniline excess is 0.05 to 10 mol %.

4. Process according to Claims 1 to 3, characterised in that the removal of at least some of the reaction water is carried out at 190 to 260° C.

5. Process according to Claims 1 to 4, characterised in that the removal of at least part of the reaction water comprises the removal of 10 to 100% of the total reaction water.

6. Process according to Claims 1 to 4, characterised in that the removal of at least part of the reaction water comprises the removal of 20 to 100% of the total reaction water.

7. Process according to Claims 1 to 6, characterised in that aniline in a molar excess of 0.5—2% and sulphuric acid in trichlorobenzene are heated under atmospheric pressure in such a manner that the total reaction water is removed at about 190 to about 210° C.

8. Process according to Claims 1 to 6, characterised in that aniline in a molar excess of 0.5—2% and sulphuric acid are heated in a closed reactor in dichlorobenzene under intrinsic pressure to about 200° C to 210° C and the total reaction water is removed at this temperature.

9. Process according to Claims 1 to 8, characterised in that the p-sulphanilic acid is obtained in the free form by filtration from the reaction mixture and subsequent drying and the filtrate thereby formed ist passed back into the reaction.

10. Process according to Claims 1 to 8, characterised in that the p-sulphanilic acid is obtained in salt form by alkaline-aqueous extraction from the reaction mixture and the remaining organic phase is passed back into the reaction, the aqueous-alkaline extract

a) being further processed as such and hence as an aqueous solution of the p-sulphanilic acid, or
b) being evaporated and the p-sulphanilic acid salt being isolated or
c) being acidified and the free p-sulphanilic acid thereby precipitated being isolated by filtering off the precipitate by suction.

**Revendications**

1. Procédé de préparation de l'acide p-sulfanilique selon le procédé dit Back par réaction d'hydrogénosulfate d'anilinium à températue élevée dans un solvant inerte, procédé caractérisé en ce qu'on effectue la réaction en présence d'un excès de l'aniline, qui s'écarte de la proportion équimolaire de l'aniline et de l'acide sulfurique, et en ce qu'on réalise au moins partiellement entre 180 et 280°C, éventuellement sous pression, l'enlèvement de l'eau de réaction du mélange réactionnel.

2. Procédé selon la revendication 1, caractérisé en ce qu'on met en réaction l'aniline en un excès d'au moins 0,01 mol %.

3. Procédé selon la revendications 1 et 2, caractérisé en ce que l'excès d'aniline est de 0,05 à 10 mol %.

4. Procédé selon la revendications 1 à 3, caractérisé en ce qu'on effectue l'élimination au moins partielle de l'eau de réaction entre 190 et 260°C.

5. Procédé selon la revendications 1 à 4, caractérisé en ce que l'éliminatino au moins partielle de l'eau de réaction correspond à 10 à 100% de la totalité de l'eau de réaction.

6. Procédé selon la revendications 1 à 4, caractérisé en ce que l'élimination au moins partielle de l'eau de réaction correspond à 20 à 100% de l'eau totale de réaction.

7. Procédé selon la revendications 1 à 6, caractérisé en ce qu'on chauffe l'aniline, en un excès molaire de 0,5 à 2%, et l'acide sulfurique dans le trichlorobenzène sous la pression atmosphérique de manière à éliminer la totalité de l'eau de réaction entre environ 190°C et environ 210°C.

8. Procédé selon la revendications 1 à 6, caractérisé en ce qu'on chauffe l'aniline, en un excès molaire de 0,5 à 2%, et l'acide sulfurique dans un réacteur clos dans du dichlorobenzène sous la pression autogène jusqu'à environ 200°C à 210°C et en ce qu'on élimine à cette température la totalité de l'eau de réaction.

9. Procédé selon la revendications 1 à 8, caractérisé en ce qu'on obtient l'acide p-sulfanilique sous forme libre par filtration du mélange réactionnel puis séchage, et en ce qu'on recycle dans la réaction le filtrat ainsi obtenu.

10. Procédé selon la revendications 1 à 8, caractérisé en ce qu'on obtient l'acide p-sulfanilique sous forme de sel par extraction alcaline aqueuse du mélange réactionnel et en ce qu'on recycle dans la réaction la phase organique restante, et en ce qu'on soumet l'extrait alcalin aqueux:

a)   àla suite du traitement, sous cette forme et donc sous forme de solution aqueuse du sel de l'acide p-sulfanilique, ou

b)   à une concentration par évaporation, et l'on recueille le sel de l'acide p-sulfanilique, ou

c)   à une acidification et l'on obtient, par filtration et essorage du précipité, l'acide p-sulfanilique libre ainsi précipité.